# EUROPEAN PATENT SPECIFICATION

(11) **EP 3 630 213 B1**
(45) Date of publication and mention of the grant of the patent: **05.07.2023**
(21) Application number: 18726495.7
(22) Date of filing: 30.05.2018
(51) Int. Cl.: A61L 27/36

(54) **CARTILAGE GRAFT SCAFFOLDS**
DEZELLULÄRISIERTES KNORPELARTIGES GERÜST
ÉCHAFAUDAGE CARTILAGINOÏDE DÉCELLULARISÉ

(30) Priority: 30.05.2017 EP 17173516
(43) Date of publication of application: 08.04.2020
(73) Proprietor: Trauma Care Consult Traumatologische Forschung Gemeinnützige Gesellschaft mbH, 1060 Vienna (AT)
(72) Inventor: REDL, Heinz, 1060 Vienna (AT); NÜRNBERGER, Sylvia, 1130 Vienna (AT); ZEHETNER, Johann, 6850 Dornbirn (AT)
(74) Representative: Gassner, Birgitta
(86) International application number: PCT/EP2018/064245
(87) International publication number: WO 2018/220047

(56) References cited:
- WO-A2-2006/090372
- WO-A2-2009/022191
- US-A1- 2008 077 251

## Description

### Field of the Invention

The present invention relates to cartilage graft scaffolds with engraved notches useful in therapeutic applications and methods for producing such scaffolds.

### Background Art

Matrix-associated chondrocyte transplantation (MACT or MACI) is currently the gold standard for the treatment of large articular cartilage defects and leads to an improvement of symptoms in most patients. However, graft failures still occur and success is highly dependent on a long post-surgery physiotherapy. While donor age, body mass index, defect type and surgical approach play a role, one significant cause of graft failure is insufficient rehabilitation or traumatic occurrence after implantation, both linked to early loading (Marlovits et al., Eur J Radiol. 2006 Jan;57(1):24-31), which could be prevented by a reduction of the timeframe where loading poses a risk or less susceptible implants, e.g., by stiffer scaffold materials.

The most natural mechanical behavior can be found in articular cartilage itself. Previous studies have shown the possibility to decellularize cartilage with tolerable impact on biomechanics, even when the glycosaminoglycans (GAG) have been depleted in the process in order to make the matrix more accessible (Schneider et al., Tissue Eng Part C Methods. 2016 Dec;22(12):1095-1107).

Some graft failures occur very late, although until then cartilage tissue formation had taken place and performed well for more than a year. Long-term follow-up clinical studies report cartilage hypertrophy and the formation of fibrocartilage in up to one third of patients with a need for reoperation in 10 percent of patients.

In addition to the improved biomechanical properties, in a decellularized scaffold the complex structure of collagen in articular cartilage has been preserved bearing the advantage of a most natural environment. Cartilage derived collagen type II has been shown to provide chondroinductive stimuli. Combined with the natural 3D architecture, this might counteract the formation of unwanted types of cartilage and thus have a positive effect on the long term functionality of the graft.

Reseeding tests have shown that cells attach well to the surface of the decellularized scaffolds but show poor infiltration of the matrix. This is a well-known challenge posed by the extremely dense hyaline cartilage ECM.

WO2006/090372 discloses a method for providing cartilage-containing tissue for grafting. Cartilage-containing tissue is excised and treated under appropriate cryogenic preservation conditions so as to yield cryogenically preserved cartilage-containing tissue. After thawing the preserved cartilage-containing tissue comprises at least 10% viable chondrocytes throughout the cartilage portion. The excised cartilage-containing tissue comprises bony tissue topped by a cartilage portion or layer. At least one or a plurality of notches may be made to the cartilage portion.

WO2014/011891 describes porated cartilage products. The cartilage products were made by isolating, porating, digesting, and cryopreserving a cartilage sample by reducing the temperature in a step-wise manner. Such treated cartilage products comprise enhanced levels of viable chondrocytes.

US2014/0243993 discloses mosaic cartilage. The mosaic cartilage comprises cartilage sheets of a plurality of interconnected cartilage tiles and a biocompatible carrier and additional biological components e.g., fibrin or collagen gel. The cartilage tiles may exhibit a plurality of channels in the cartilage tissue. The perforations also allow for enhanced cryopreservation of cartilage constructs or sheets. The viable chondrocytes within the cartilage sheet can migrate out of the sheet and carry out repair and regenerative functions.

WO2009022191 describes articular cartilages for repairing cartilage defects which are made from pure cartilage and have incisions on the surface facing the bone. The incisions are provided by cutting blades. The cartilage with incisions may be seeded with cells on the surface.

The cartilage products according to the state of the art comprise viable and/or dead chondrocytes. However, the cellular components could be an antigenic stimulus for alloreactive immune response. Therefore, there is still the need for scaffolds with mechanical characteristics similar to normal cartilages which do not evoke alloreactive immune responses.

### Summary of invention

Thus it is an object of the invention to provide a cartilage graft scaffold with engraved incisions for cells to grow in, thus enabling scaffold reseeding and optimizing graft integration and which does not evoke an alloreactive immune response.

The object is solved by the subject matter of the present invention.

The present invention relates to a biomaterial comprising a cartilage graft scaffold comprising less than 5% viable cell material, wherein the cartilage graft scaffold is depleted of glycosaminoglycans (GAG) and comprises on the surface a plurality of notches in form of lamellae or grids, wherein the notches do not perforate the cartilage graft scaffold.

According to the invention the notches do not perforate the cartilage graft scaffold. In some embodiments additional holes may perforate the cartilage graft scaffold in order to allow flow of fluids from one side to the other.

According to further embodiments of the invention the notches are of a defined depth, width and/or distance. Specifically, the notches have a depth of about 20 to 5.000 µm, or about 20 to 3.000 µm, or about 20 to 1.000 µm, or about 20 to 500 µm, or about 20 to 50 µm. In some embodiments of the inventions the distance between the notches is about 10 to 1.000 µm, or about 10 to 100 µm, or about 10 to 50 µm.

According to the claimed invention, the notches are in form of lamellae or grids.

The lamellae form of the notches is specifically suited for providing space for cells to penetrate deep into the cartilage graft scaffold and deposit new matrix therein. Due to the removal of matrix components, such as the removal of glycosaminoglycans (GAG) which is according to the present invention, the adhesion of the cells is improved and forming of new tissue facilitated.

A further embodiment of the invention relates to the biomaterial as described herein, wherein the cartilage graft scaffold has less than 2 %, or less than 1 % viable chondrocytes throughout the cartilage graft scaffold.

Specifically, the cartilage graft scaffold is selected from the group consisting of elastic cartilage, or hyaline cartilage fibrocartilage.

According to a further embodiment of the invention the cartilage graft scaffold is preseeded. Specifically, the cartilage graft scaffold is preseeded with chondrocytes, or other cells with chondrogenic potential (e.g., mesenchymal stem cells, bone marrow cells) of autologous or allogenic origin.

In a further embodiment, the cells may be added to the scaffold at time of implantation. The cells may be selected from the group consisting of cells with chondrogenic potential ( chondrocytes, mesenchymal stem cells, bone marrow cells). One embodiment of the invention relates to a method for preparing a biomaterial, comprising the steps of
a. obtaining a cartilage layer from a donor,
b. incising a plurality of notches on the surface, wherein the notches do not perforate the cartilage graft scaffold,
c. decellularizing and/or devitalizing the cartilage biomaterial, and
d. depleting the cartilage biomaterial of glycosaminoglycans.

A further embodiment of the invention relates to the method as described herein, wherein the incision are conducted with a laser beam. Specifically the notches are provided by a CO₂ or a femtosecond laser.

In some embodiments viable cells (e.g., chondrocytes) are removed from the cartilage graft scaffold. Specifically, the cells are chemically devitalized, e.g., by acidic or alkaline treatments, ionic detergents, non-ionic detergents, and zwitterionic detergents treatments.

In some embodiments viable cells of the cartilage graft scaffold are devitalized. Specifically the cells are damaged by physical methods, e.g., by freeze/thaw cycles. The most common physical methods used to damage and partially remove cells from the matrix of a tissue are based on the use of temperature, force and pressure, and electrical disruption.

One embodiment of the invention relates to the biomaterial as described herein for use in tissue regeneration or for treating osteochondral defects.

A further embodiment of the invention relates to the biomaterial as described herein for use in treating a cartilage bone or muscle defects.

### Brief description of drawings

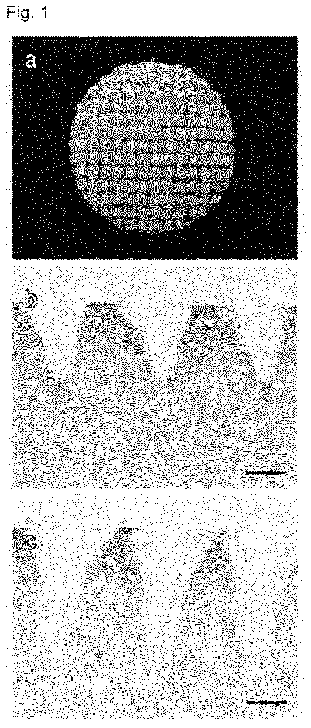

Fig. 1 depicts CO₂-laser lamella in cartilage performed with varying speed and repetitions. (a) The macroscopic view shows the regular grid pattern in the 8 mm cartilage biopsy performed with one laser setting. (b, c) The histological cross-sections show the depth of notches achieved with two or five runs. The lack of collagen type II immunostaining at the laser edges reveals the heat damage of the laser. (scale bar = 200 µm)

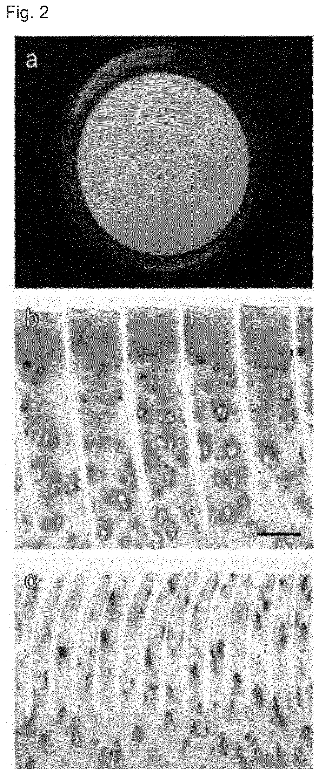

Fig. 2 depicts femtosecond-laser-engraved lines in a 8 mm cartilage biopsy (a) in macroscopic view and (b, c) collagen type II immunostained histological cross-sections of the cartilage graft scaffold. (b: 1000 runs, 0.25 mm distance; c: 600 runs, 0.1 mm distance, 1 m/sec; scale bar = 200 µm)

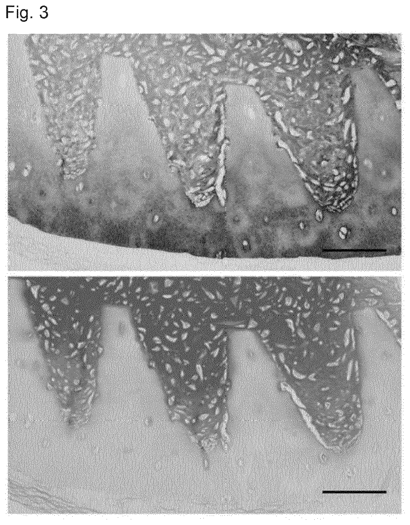

Fig. 3 depicts CO₂ laser-engraved cartilage graft scaffolds, decellularized and GAG-depleted and seeded with ASCs and cultivated in chondrogenic differentiation medium for five weeks; Collagen type II and Alcian blue stained histological paraffin sections show the well differentiated tissue that formed in between the lamella and that integrated well with the cartilage graft scaffold (scale bar = 200 µm).

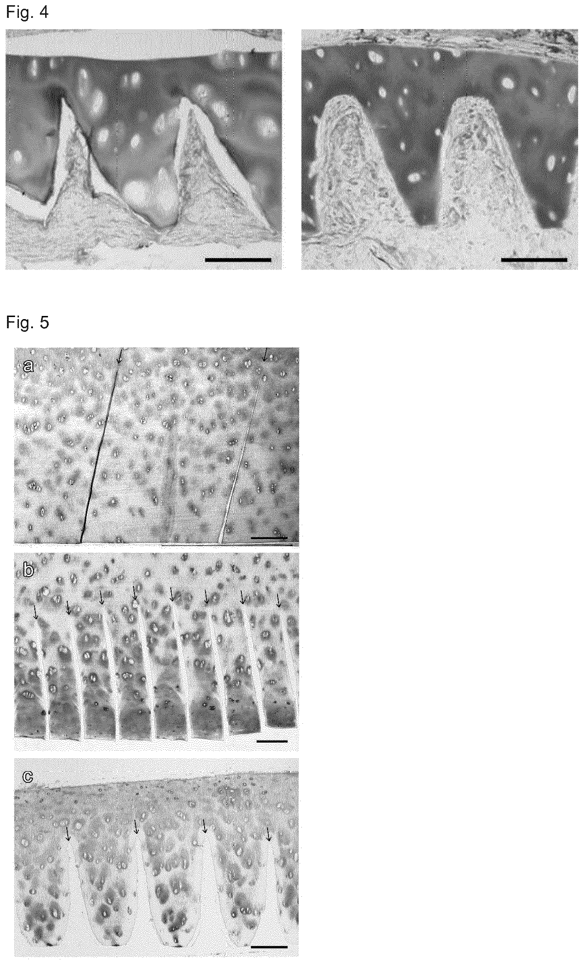

Fig. 4 left: devitalized lamellar graft (freeze-thaw cycles) filled with repair tissue which however detaches from the cartilage surface, right: On pretreated lamellar graft (HCl, pepsin and NaOH) the newly formed tissue densely attaches to the cartilage edge of the scaffold (scale bar = 200 µm).
Fig. 5 depicts notches in cartilage produced with different techniques a) Razor blade incision with the edges of the cartilage close together b) Femtosecond laser notches generating a space of about 50µm in between the cartilage edges of the lamella. c) CO₂ laser produce most material ablation leading to V-shaped notches (scale bar = 200 µm).

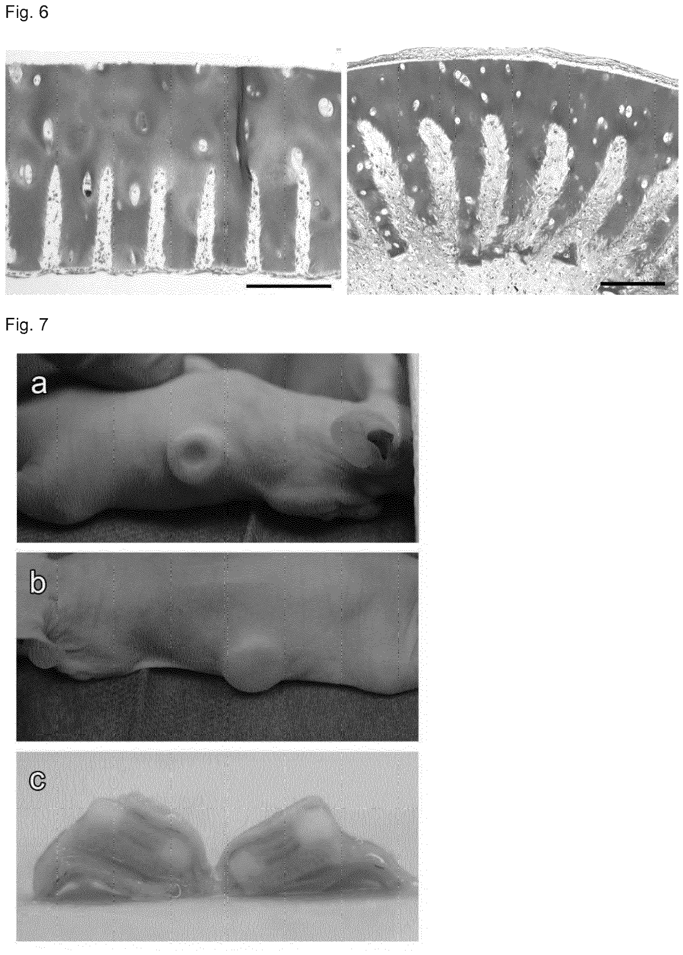

Fig. 6 shows reseeded femtosecond lasered scaffold with a low amount of cells in the lamella space (left) leading to a repair tissue over time (right) (scale bar = 200 µm).
Fig. 7 depicts nude mouse with subcutaneously implanted osteochondral plugs with (a) an unfilled defect; (b) Defect filled with reseeded scaffold. (c) Explanted osteochondral plug after 5 weeks defects with reseeded scaffolds are filled with tissue.

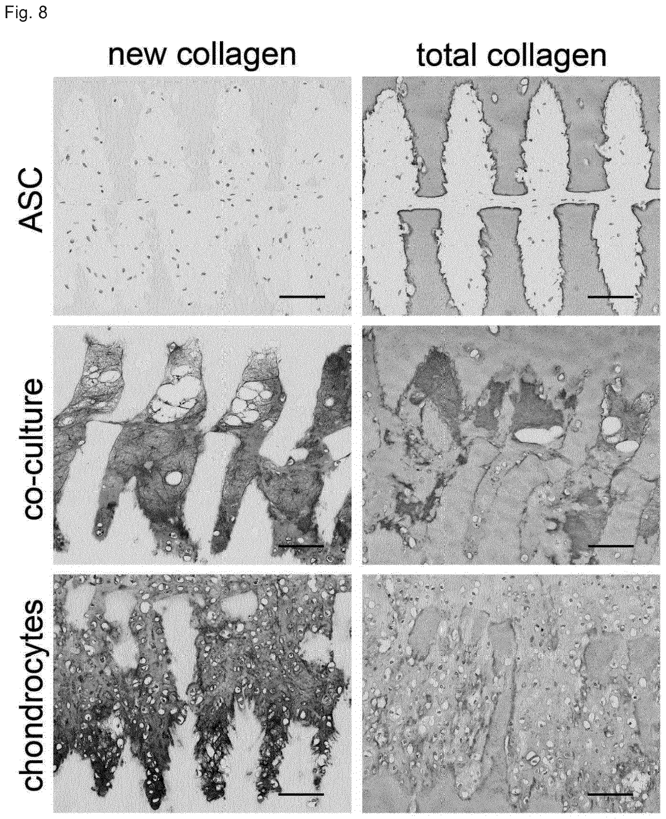

Fig. 8 depicts *in vivo* results of FS-laser engraved scaffolds seeded with ASCs, co-culture and chondrocytes show tissue formation of the seeded cells in between the lamella and good integration of the newly formed tissue with the scaffold. Paraffin sections were immune-stained with an antibody reacting with newly synthesized collagen type II (left row) and total collagen types II (right row) showing collagen type II deposition of chondrocytes and co-cultures. (scale bar = 100 µm)

### Description of embodiments

Surprisingly it was found that a cartilage graft scaffold with engraved incisions (notches) for cells to grow in is a promising option to enable scaffold reseeding and optimize graft integration.

Thus, provided herein is a biomaterial comprising a decellularized cartilage graft scaffold, wherein the cartilage graft scaffold comprises a plurality of notches.

As used herein, the term "cartilage graft scaffold" refers to any tissue, specifically to natural cartilage tissue.

Cartilage is an avascular connective tissue made up of collagen and/or elastin fibers, and chondrocytes, all of which form a matrix and may contain further components such as glycosaminoglycans. A natural cartilage graft scaffold may be obtained from any source, such as for example, hyaline cartilage, such as the trachea, ribs or articular cartilage present at the end of joints, such as knee, hip, shoulder, elbow, etc., or fibrocartilage, such as cartilage present in the pubic symphysis, the annulus fibrosus of intervertebral discs, menisci and the temporomandibular joints (TMJ), or from elastic cartilage, such as cartilage present in the outer ear, Eustachian tube and epiglottis. Hyaline cartilage can be found on the ends of bones which form joints, on the ends of the ribs, on the end of the nose, on the stiff rings around the windpipe, and supporting the larynx. Articular cartilage is a specialized type of hyaline cartilage which covers the surface of joints and provides a durable low friction surface that distributes mechanical forces and protects the joint's underlying bone. Fibrocartilage is found between the bones of the spinal column, hips and pelvis.

The cartilage graft scaffold may be obtained from a human or animal donor, a juvenile donor, a deceased individual (animal or human, i.e. cadaver). Specifically, there are many joints on the human body which may serve as harvest sites for producing hyaline/articular cartilage graft scaffolds. Examples of joints in the foot that could be used to harvest articular cartilage graft scaffold include, but are not limited to, the calcaneal-cuboid joint, intercuneiform joints, tarsometatarsal joints, lesser metatarsophalangeal joints, interphalangeal joints, and hip joint, e.g., from the femoral head.

According to a further embodiment the cartilage graft scaffold is a human cartilage graft scaffold. According to a further embodiment the cartilage graft scaffold is a non-human scaffold. In a further embodiment the cartilage graft scaffold is an articular cartilage.

The cartilage graft scaffold may have a thickness of about 100 µm to 5.000 µm. Typically the thickness may be between about 250 µm and 3.000 µm. The thickness of the cartilage graft scaffold may be uniform or non-uniform. Preferably the thickness is uniform.

As used herein, the term "decellularized" refers to a biomaterial which is substantially free of viable cellular material, e.g., substantially free of viable chondrocytes.

The phrase "substantially free" as used herein refers to a cartilage graft scaffold produced by the invention wherein at least 95 % of the viable cellular material has been devitalized and/or removed from the scaffold, preferably about 96 %, 97 %, 98 %, 99 %, 99.5 %, 99.8%, 99.9 % or 100% of the viable cellular material have been devitalized and/or removed.

"Decellularized cartilage graft scaffold" means cartilage graft scaffold substantially free of cellular components to eliminate or reduce antigenicity of the cartilage graft scaffold to an extent where the scaffold would be considered non-immunogenic as a xenograft.

Decellularization results in an acellular matrix that has low immunogenicity with the same biochemical make-up as native cartilage. Devitalized cartilage (DVC), on the other hand, results in a matrix which may still contain cell residuals and antigenic cell surface markers. For decellularization typically physical and chemical processes are employed, whereas for devitalization physical methods are used. Successful decellularization of cartilage may be accomplished using different methods with differing results with respect to the remaining biochemical content, cell removal, and mechanical performance, e.g., using reciprocating osmotic shock, detergents, and enzymatic washes. Devitalization of a cartilage graft scaffold may be carried out by freezing/thawing cycles, or freezing at -20 ° C.

One embodiment relates to a biomaterial comprising a decellularized cartilage graft scaffold, wherein the scaffold comprises a plurality of incisions or notches. The plurality of incisions or notches can be at a depth of between about 5 and about 95 % of the thickness of the biomaterial. The plurality of incisions can be of non-uniform depth and width. Additionally, the plurality of incisions or notches can be arranged in either a uniform or non-uniform array.

The plurality of incisions or notches is provided in an incision pattern over the scaffold. The incision pattern may be a plurality of elongated channels, either straight or curved, in substantially parallel spaced relationship, a plurality of elongated channels radiating from a common central area, a plurality of concentric channels radiating from a common central area; a plurality of elongated channels forming a grid; a plurality of elongated channels forming a "zig zag" pattern, a plurality of elongated channels forming a wave-like pattern, e.g., lamellar, a plurality of elongated channels forming a spiral pattern, a plurality of mutually-spaced point notches arranged in a suitable dimensional matrix, or any combinations thereof as well as many other patterns as envisaged by the person skilled in the art.

A further embodiment relates to a biomaterial comprising a decellularized cartilage graft scaffold having a plurality of notches, wherein the notches are engraved on the surface of the cartilage graft scaffold. The notches may have a predetermined depth. The predetermined depth of the incision may be 20 µm from the surface of the cartilage graft scaffold, or even 25 µm, 50 µm, 75 µm, 100 µm, 150 µm, 200 µm, 250 µm, 500 µm, 750 µm, 1.000 µm, 1.500 µm, 2.000 µm, 2.500 µm, 3.000 µm, 3.500 µm, 4.000 µm, 4.500 µm, or 5.000 µm from the surface, or even deeper. It is to be understood that the predetermined depth does not exceed the thickness of the cartilage graft scaffold, i.e. does not perforate the cartilage graft scaffold.

In some embodiments local perforations may be provided in order to allow fluid flow from one side to the other.

By way of example, the width of each incision may be about 15 µm to about 25 µm, or of about 25 µm to about 100 µm. However, the width may be less than about 15 µm or even greater than about 100 µm. The distance between adjacent notches, for example between adjacent notches, between concentric notches, or between adjacent linear notches or linearly arranged notches, may be of about 10 µm, 50 µm 100 µm, 250 µm, 500 µm, 750 µm, or 1.000 µm. The depth of the notches may be substantially uniform across the cartilage layer, or alternatively may vary from notch to notch.

A further embodiment relates to an automatized approach, which not only offers high precision and standardization, but also allows for large scale production of off-the-shelf biomaterials.

Notches performed with cutting devices such as razor blades or knives lead to cutting edges that lie close together with almost no fillable space in between (Fig. 5a). These notches do only open up if they are bended (or due to artefacts such as it may be the case during histological processing).

However, a certain gap is necessary to provide space for the cells, for reseeding, to avoid mechanical damage and for tissue formation. Lasers ablate material during the incisioning, the amount of removed material depending on the laser type and technology. Femtosecond lasers allow narrow notches (e.g. 50 µm) (Fig. 5b), which are however still broad enough to allow repopulation with cells and provide a protected environment for tissue formation. Notches produced by FS laser can be considered as the minimal required space for successful tissue generation (Fig. 6). CO₂ laser generate V-shaped notches enabling almost a 1:1 ratio of cartilage scaffold matrix and the newly formed tissue (Fig. 5c).

The notches may be incisions, cuts, apertures, holes, gaps, channels, fissures, and the like, and may be arranged in any suitable pattern as described herein. The notches may be performed by variety of means, e.g., milling cutters or laser beams. In one embodiment of the invention the notches are produced by a laser beam. By using a laser, not only notches are made but also defined amount of the cartilage matrix is locally removed. Thus, in a preferred embodiment, the notches are produced by a laser beam and thereby matrix components are locally removed. According to the claimed invention, glycosaminoglycans are selectively removed from the cartilage matrix.

According to one embodiment a carbon dioxide laser to generate fine well-defined structures is used. According to another embodiment of the invention a femtosecond laser is used. Laser engraving may be performed in repetitions. Depending on the depth of the notches, one, two, three, four, five, or more repetitions are performed.

By using laser created notches the cells can be lead deep into the scaffold where they fill up the notches, form new cartilage and support graft remodeling, including the restoration of the GAG content, from throughout the whole defect depth. Suitable cells are either chondrocytes or other cells with chondrogenic potential (e.g., stem cells) of autologous or allogenic origin. They are either preseeded on the scaffold before implantation (either intraoperatively or in the cell culture lab in case of pre-cultivation), or combined with the scaffold directly inside the defect by applying a cell suspension or bone marrow stimulation.

Moreover, the cells might be stimulated to infiltrate the decellularized matrix as soon as the notches are full and their only option is to migrate sideways in between the collagen sheets.

According to one embodiment of the invention a laser can be used to engrave fine structures into cartilage graft scaffolds, e.g., articular cartilage, and those structures can be filled with cells to achieve a well-integrated differentiated tissue. The production of laser engraved decellularized scaffolds is precise, reproducible and can be up-scaled with hardly any rise in effort or time.

CO₂ lasers emit a beam of infrared light with a wavelength of 10.6 µm in a laser medium consisting of carbon dioxide, nitrogen and helium. A variety of adjustable parameters such as energy and velocity (whose product equals the laser power), beam diameter (and with it energy density and irradiance), pulse duration and repetition rate give rise to a multitude of options to achieve the desired engraving depth and limit thermal damage. Increased power, long pulse durations as well as decreased speed result in deeper engraving or cutting. As ablation is thermal in lasers with laser pulse length exceeding 10 psec (e.g. CO₂, but not in ultrashort-pulsed lasers as femtosecond-lasers), ECM molecules of the remaining tissue might be damaged in the process. During prolonged exposure to heat, the triple helical structure of native collagen is transformed into a random coil by destabilization of hydrogen bonds and Van-der-Waals forces. Immunohistochemistry showed changes in the collagen type II structure at the lasered edges in relation to these parameters. This unwanted side effect can be minimized by avoiding thermal energy deposition into the non-ablated tissue, either by sufficiently short exposition of the tissue or by use or femtosecond-laser when the velocity of the tissue ablation front is similar to or faster than the heat diffusion into the residual tissue (cold ablation).

In some embodiments, the above mentioned disadvantage may be used for an alternative method to provide notches in cartilage. The cartilage graft scaffold is locally exposed to prolonged exposure to heat in order to transform collagen into gelatin. The formed gelatin is then removed, thereby local notches are obtained. The treatment may be performed by heat generating or heat-transferring instruments.

Examples of heat generating or heat-transferring instruments include, but are not limited to, lasers, heated scalpel blade, heated scissors or heated forceps. The instrument should be heated to a temperature of about 200 ° C. In another embodiment, heat may be applied by a CO₂, or femtosecond laser.

Therefore, a further embodiment of the invention relates to laser settings with selected suitable values of energy and velocity to optimize the results. Short exposition time by choosing a fast laser movement and short pulse duration are preferred. Incision depths are determined by a repetition rate or due prolonged exposure.

Compared to holes, the lamella structures bear the advantage of making the scaffold more flexible, potentially maximizing the cell-scaffold contact surface and scaffold-host contact in any defect. They provide more space for new matrix to be generated and may enable a laminar flow of medium through the notches in the early days of cultivation while they are not yet filled by newly generated matrix. The native structure of the collagen is preserved in the lamellae or pillars (except of the laser or cutting edges) and the newly synthesized matrix is nudged towards a vertical matrix alignment by the scaffold's topography - an advantage over sponge grafts which promote circular matrix deposition. Confocal microscopy showed the collagen fibers to be aligned along the cutting edges and perpendicular to the cartilage superficial zone inside the notches, while close to the superficial zone where the notches are broader the matrix is more unaligned with a tendency to align along the scaffold surface - both characteristics of the collagen network of articular cartilage.

On devitalized (but not decellularized and not GAG-depleted) scaffolds, which are not according to the claimed invention, histology revealed a gap between the newly formed matrix and the scaffold. This might be due to shrinking which occurred in course of the histological sample preparation, but nevertheless means the new cells/tissue adhered less tightly to the scaffold surface than in decell-deGAG samples, which are according to the claimed invention, where this effect was not observed (Fig. 4).

In order to further improve the adhesion of cells in GAG-depleted cartilage graft scaffolds, the scaffolds may be treated with chemicals. In one embodiment of the invention the cartilage graft scaffold is treated with an enzyme, an acidic and/or a basic solution. In a preferred embodiment, the cartilage graft scaffold is treated with pepsin, HCl and NaOH.

For devitalization the cartilage samples are kept at about -20 ° C and room temperature for about 1 hour respectively, several times frozen dryly and several times frozen submerged in hypotonic buffer. Then the cartilage samples are incubated in hydrochloric acid overnight for decellularization, in an enzyme solution overnight for selective matrix depletion, followed by incubation in sodium hydroxide to roughen the edges.

The cartilage graft scaffold can be used to treat subjects in need thereof. It is envisaged that the biomaterial comprising a decellularized and/or devitalized cartilage graft scaffold can facilitate or enhance the accurate matrix deposition and tissue formation. The plurality of notches on the surface of the cartilage graft scaffold creates a construct having increased surface area relative to a cartilage lacking said notches. The increased surface area can allow cell adhesion and nutrients to transfuse easily within, throughout and across the cartilage graft scaffold at the injury site and thus may contribute to enhanced regeneration or healing.

The biomaterials of the invention are useful for the repair, replacement and regeneration of cartilage and cartilage tissue. A cartilage graft scaffold may be defined as dense connective tissue layer comprised of proteoglycan and collagen.

The inventive approach generates scaffolds with superior mechanical properties compared to commercially available scaffolds currently used in cartilage regeneration and positive effects on cell adhesion and matrix deposition. The laser generated structures promote enhanced graft integration and faster remodeling by greatly increasing the cell-scaffold contact and the amount of cells in relation to cartilage matrix by retained mechanical properties. It is envisaged that decellularized GAG-depleted cartilage with engraved lamella and grid-patterns has the potential to reduce the duration of post-surgery physiotherapy and the risk of graft failure, thus being beneficial for health funds by reducing treatment costs as well as patients by enabling a faster return to their daily lives.

A further embodiment of the invention relates to the biomaterial as described herein which is applied to a site within a patient for treating cartilage and/or bone defects. For example, cells from surrounding tissue can reproduce and generate new cartilage *in vitro.* The newly formed cartilage tissue can fill defects and integrate with existing native cartilage and/or subchondral bone at the treatment site.

According to a further embodiment of the invention the biomaterial may be administered at a site of defect in cartilage, bone, ligament, tendon, meniscus, joint, or muscle. Further described herein is the biomaterial for use in the treatment of degenerative defects or injuries. Further described herein is the biomaterial for use in the treatment of osteoarthritis or muscle defects.

Specifically, the biomaterial may be administered to a subject to repair cartilage, or to promote cartilage grow or for regeneration. The biomaterial may be applied to a joint (e.g., knee), to bone (e.g., femur or humerus), or to cartilage.

Specifically, the biomaterial may be administered to a subject having soft tissue defect, for the repair and/or regeneration thereof. The biomaterial may be applied to a ligament, tendon, or muscle. The soft tissue defect may be a sprain, strain, contusion, or stress injury to a ligament, tendon, or muscle.

Specifically, the biomaterial may be administered locally to the subject in need thereof. The biomaterial may be surgically implanted, preferably the biomaterial is administered in a minimally invasive procedure, e.g., by arthroscopy.

### Examples

The examples which follow are set forth to aid in the understanding of the invention but are not intended to, and should not be construed to limit the scope of the invention in any way. The examples do not include detailed descriptions of conventional methods. Such methods are well known to those of ordinary skill in the art.

### MATERIAL and METHODS

### Sample harvest

Macroscopically intact human articular cartilage was harvested from femoral heads of donors undergoing hip replacement, with patient's consent and the approval of the local ethical board. Full thickness non-calcified cartilage was separated from the subchondral bone and biopsies prepared with a biopsy punch of 8 mm in diameter and washed with PBS + antibiotics (Pen/Strep). Cartilage biopsies were used in full thickness or sample thickness standardized to 300 µm using the Teixido cartilage cutter (MicroFrance^{®} by Integra LifeSciences, USA). Cartilage was taken from the middle zone of biopsies, tissue from the superficial zone and the deep calcified zone was discarded.

### Laser Settings

Laser-engraving was either performed with a CO₂-laser or a femtosecond-laser. The former was a Trotec Speedy 300 (Trotec Ltd, Austria) with a standard wavelength of 10.6 µm, constantly set on pulse duration of 0.2 msec. The femtosecond-laser was a Spirit^{®} High Q Laser used with a wavelength of 520 µm and 100 mm focal distance. To pierce holes into the cartilage biopsies, the CO₂-laser was set to 30 W and 5000 Hz and the speed to 42.6 cm/s, one to five line-patterns were performed. The femtosecond-laser was constantly set to 12.4 J/cm², equaling 7 µJ, and a pulse repetition rate of 200 kHz. Full thickness cartilage biopsies were engraved in varying intervals with 400-600 runs and 1000 mm/s. Cartilage biopsies with 300 µm thicknesses were engraved with 140 runs.

To generate a grid pattern, the laser beam was translated in parallel horizontal and vertical lines across the samples for the femtosecond-laser and for the CO₂ laser the sample was rotated for 90° and the parallel line pattern repeated.

### Devitalization / Decellularization

Cartilage biopsies with laser-engraved grid patterns were either devitalized or decellularized and subjected to targeted matrix depletion ("decell-deGAG").

For devitalization samples were alternately kept at -20 ° C and room temperature for 1 hour respectively, twice frozen dryly and twice frozen submerged in hypotonic buffer (10 mM Tris-base, adjusted to pH 8.0 using HCl). Decell-deGAG samples also underwent these freeze/thaw cycles, then were incubated in 0.1 M hydrochloric acid overnight for decellularization, in 1 mg/mL pepsin (0.1% in 0.5 M acetic acid) overnight for selective matrix depletion, followed by a 6 hour incubation in 0.1 M sodium hydroxide to roughen the edges. All incubation steps took place at 37 ° C under continuous shaking.

### Cell culture

Human adipose-derived stromal/stem cells (ASCs) were harvested via trypsinization and used for the reseeding experiments.

### In vivo study

A small animal model was chosen to prove the behavior of the scaffold materials in a cartilage environment and under systemic conditions. Bovine osteochondral plugs were implanted subcutaneously into the nude mice. The osteochondral plugs (1 cm diameter) bore experimental cartilage defects (4 mm). These defects served as knee-defect-model and were filled ("treated") with the developed biomaterials (a stack of two 300 µm chips) with 1×10⁶ cells (human ASCs, bovine chondrocytes or co-cultures). An empty defect served as control. The cells were preseeded two days before implantation. Samples were taken 6 weeks after implantation.

### Histological examination

*In vitro* and *in vivo* samples were fixed in 4 % neutral buffered formalin, embedded in paraffin and stained with Alcian blue (pH 2,5) and a collage type II antibody.

### RESULTS

### Laser settings

CO₂-laser engravings showed a V-shaped incision. The depth is regular within a specific setting and increased with the number of repetitions ranging from 150 µm at one repetition to 680 µm at five repetitions. The laser edges show collagen type II denaturation, which was about 50 µm wide (Fig. 1). Femtosecond-laser engraving produced straight and parallel notches with narrow distances between the lamella. The surface of the lamella does not show any alteration of the matrix (Fig. 2)

### In vitro performance of the scaffolds

Reseeding of CO₂-laser engraved cartilage biopsies with ASCs resulted in a coherent construct of the scaffold and a chondrogenic-differentiated tissue produced de-novo by ASCs. The ASCs deposited collagen type II and proteoglycans in their intracellular space and filled up the lamella with this matrix. The cells and the matrix integrated well with the scaffold by complete and intense adhesion to the surface (Fig. 3).

### In vivo performance of the scaffolds

The implantation of the cell-seeded scaffolds into an experimental defect showed that the material performed well in a cartilage environment under systemic conditions of the nude mouse. It persisted almost unaltered over the six weeks and completely filled the defect. The difference to the control was visible even macroscopically under the skin of nude mice or when the cylinder was cut into half after explanation (Fig. 7). Six weeks after of *in vivo* implantation, the scaffold was still present and well embedded in newly formed tissue. Tissue formed by ASCs did not show signs of chondrogenic differentiation, ASCs in co-culture with chondrocytes and chondrocytes alone formed cartilage tissue. It was positive for collagen type II, had a dense appearance and complete adhesion and integration with the scaffold matrix (Fig. 8)

## Claims

1. A biomaterial comprising a cartilage graft scaffold comprising less than 5% viable cell material, wherein the cartilage scaffold is depleted of glycosaminoglycans (GAG) and comprises on the surface a plurality of notches in form of lamellae or grids, wherein the notches do not perforate the cartilage graft scaffold.

2. The biomaterial according to claim 1, wherein the notches are of a defined depth, width and/or distance.

3. The biomaterial according to claim 2, wherein the notches have a depth of 20 to 5.000 µm, or of 20 to 3.000 µm, or of 20 to 1.000 µm, or of 20 to 500 µm, or of 20 to 50 µm, and wherein the distance between the notches is 10 to 1.000 µm, or 10 to 100 µm, or 10 to 50 µm.

4. The biomaterial according to any of claim 1 to 3, wherein the cartilage graft scaffold has less than 2 %, or less than 1 % viable cell material.

5. The biomaterial according to any of claim 1 to 4, wherein the cartilage graft scaffold is selected from the group consisting of elastic cartilage and hyaline cartilage fibrocartilage.

6. The biomaterial according to claim 1 to 5, wherein cells are added to the cartilage graft scaffold at the time of implantation, or wherein the cartilage graft scaffold is preseeded.

7. The biomaterial according to claim 5 or 6, wherein the cells for preseeding or for addition to the scaffold at time of implantation are selected from the group consisting of chondrocytes, mesenchymal stem cells, bone marrow cells.

8. A method for preparing a biomaterial, comprising the steps of
a. obtaining a cartilage layer from a donor,
b. incising a plurality of notches on the surface, wherein the notches do not perforate the cartilage graft scaffold,
c. decellularizing and/or devitalizing the cartilage biomaterial, and
d. depleting the cartilage biomaterial of glycosaminoglycans.

9. The method according to claim 8, wherein the notches are conducted by a laser beam, and optionally the laser is a femtosecond laser or a CO₂ laser.

10. The method according to claims 8 or 9, wherein the biomaterial is pretreated with a pretreatment solution comprising HCl, NaOH, and 0.1 to 10 U/mL pepsin.

11. A biomaterial comprising a decellularized cartilage scaffold according to any of claims 1 to 7, for use in tissue regeneration or for treating osteochondral defects.

12. The biomaterial according to claim 11, for use in treating cartilage, bone, or muscle defects.

## Patentansprüche

1. Biomaterial, umfassend ein Knorpeltransplantat-Gerüst, umfassend weniger als 5 % lebensfähiges Zellmaterial, wobei das Knorpelgerüst an Glycosaminoglycanen (GAG) abgereichert ist und an der Oberfläche eine Vielzahl von Kerben in Form von Lamellen oder Gittern umfasst, wobei die Kerben das Knorpeltransplantat-Gerüst nicht durchdringen.

2. Biomaterial nach Anspruch 1, wobei die Kerben eine definierte Tiefe, Breite und/oder einen definierten Abstand aufweisen.

3. Biomaterial nach Anspruch 2, wobei die Kerben eine Tiefe von 20 bis 5.000 µm, oder von 20 bis 3.000 µm, oder von 20 bis 1.000 µm, oder von 20 bis 500 µm, oder von 20 bis 50 µm aufweisen, und wobei der Abstand zwischen den Kerben 10 bis 1.000 µm, oder 10 bis 100 µm, oder 10 bis 50 µm beträgt.

4. Biomaterial nach einem der Ansprüche 1 bis 3, wobei das Knorpeltransplantat-Gerüst weniger als 2 %, oder weniger als 1 % lebensfähiges Zellmaterial aufweist.

5. Biomaterial nach einem der Ansprüche 1 bis 4, wobei das Knorpeltransplantat-Gerüst ausgewählt ist aus der Gruppe bestehend aus elastischem Knorpel und Hyalinknorpel-Faserknorpel.

6. Biomaterial nach Anspruch 1 bis 5, wobei Zellen zum Zeitpunkt der Implantation zu dem Knorpeltransplantat-Gerüst zugegeben werden, oder wobei das Knorpeltransplantat-Gerüst vorbesiedelt wird.

7. Biomaterial nach Anspruch 5 oder 6, wobei die Zellen für die Vorbesiedelung oder für die Zugabe zu dem Gerüst zum Zeitpunkt der Implantation aus der Gruppe ausgewählt sind, die aus Chondrozyten, mesenchymalen Stammzellen und Knochenmarkzellen besteht.

8. Verfahren zur Herstellung eines Biomaterials, umfassend die Schritte
a. Erhalten einer Knorpelschicht von einem Spender,
b. Einschneiden einer Vielzahl von Kerben an der Oberfläche, wobei die Kerben das Knorpeltransplantat-Gerüst nicht durchdringen,
c. Dezellularisieren und/oder Devitalisieren des Knorpel-Biomaterials, und
d. Abreichern von Glycosaminoglycanen aus dem Knorpel-Biomaterial.

9. Verfahren nach Anspruch 8, wobei die Kerben mittels eines Laserstrahls ausgeführt werden, und der Laser optional ein Femtosekunden-Laser oder ein CO₂-Laser ist.

10. Verfahren nach den Ansprüchen 8 oder 9, wobei das Biomaterial mit einer Vorbehandlungslösung umfassend HCl, NaOH und 0,1 bis 10 U/ml Pepsin vorbehandelt wird.

11. Biomaterial, umfassend ein dezellularisiertes Knorpelgerüst nach einem der Ansprüche 1 bis 7, zur Verwendung bei der Geweberegeneration oder zur Behandlung osteochondraler Defekte.

12. Biomaterial nach Anspruch 11, zur Verwendung bei der Behandlung von Knorpel-, Knochen- oder Muskeldefekten.

## Revendications

1. Biomatériau comprenant un support de greffe de cartilage comprenant moins de 5 % de matériau cellulaire viable, dans lequel le support de cartilage est appauvri en glycosaminoglycanes (GAG) et comprend en surface une pluralité d'encoches sous la forme de lamelles ou de grilles, dans lequel les encoches ne perforent pas le support de greffe de cartilage.

2. Biomatériau selon la revendication 1, dans lequel les encoches sont d'une profondeur, largeur et/ou distance définies.

3. Biomatériau selon la revendication 2, dans lequel les encoches ont une profondeur de 20 à 5 000 um, ou de 20 à 3 000 um, ou de 20 à 1 000 um ou de 20 à 500 um, ou de 20 à 50 um, et dans lequel le distance entre les encoches est de 10 à 1 000 um, ou 10 à 100 µm ou 10 à 50 µm.

4. Biomatériau selon l'une quelconque des revendications 1 à 3, dans lequel le support de greffe de cartilage comporte moins de 2 % ou moins de 1 % de matériau cellulaire viable.

5. Biomatériau selon l'une quelconque des revendications 1 à 4, dans lequel le support de greffe de cartilage est choisi dans le groupe constitué par un cartilage élastique et un fibrocartilage à cartilage hyalin.

6. Biomatériau selon l'une quelconque des revendications 1 à 5, dans lequel des cellules sont ajoutées au support de greffe de cartilage au moment de l'implantation, ou dans lequel le support de greffe de cartilage est préensemencé.

7. Biomatériau selon la revendication 5 ou 6, dans lequel les cellules destinées à être préensemencées ou destinées à être ajoutées au support au moment de l'implantation sont choisies dans le groupe constitué par les chondrocytes, les cellules souches mésenchymateuses, les cellules de moelle osseuse.

8. Procédé de préparation d'un biomatériau, comprenant les étapes de
a. obtention d'une couche de cartilage provenant d'un donneur,
b. incision d'une pluralité d'encoches en surface, dans laquelle les encoches ne perforent pas le support de greffe de cartilage.
c. décellularisation et/ou dévitalisation du biomatériau de cartilage, et
d. appauvrissement du biomatériau de cartilage en glycosaminoglycanes.

9. Procédé selon la revendication 8, dans lequel les encoches sont effectuées par un faisceau laser, et éventuellement le laser est un laser femtoseconde ou un laser CO₂.

10. Procédé selon la revendication 8 ou 9, dans lequel le biomatériau est prétraité avec une solution de prétraitement comprenant du HCl, du NaOH et 0,1 à 10 U/ml de pepsine.

11. Biomatériau comprenant un support de cartilage décellularisé selon l'une quelconque des revendications 1 à 7, destiné à être utilisé dans la régénération de tissus ou pour traiter des défauts ostéochondraux.

12. Biomatériau selon la revendication 11, destiné à être utilisé dans le traitement de défauts cartilagineux, osseux ou musculaires.
